# EUROPEAN PATENT APPLICATION

(11) **EP 2 502 647 A1**
(43) Date of publication of application: **26.09.2012**
(21) Application number: 12163646.8
(22) Date of filing: 17.02.2006
(51) Int. Cl.: A61N 5/06

(54) **Light therapy device for treatment of bone disorders and biostimulation of bone and soft tissue**

(30) Priority: 16.02.2006 US 355583; 17.02.2005 US 653828 P; 05.08.2005 US 705753 P
(62) Divisional of application: 06710427.3
(71) Applicant: Biolux Research Limited, Vancouver, British Columbia V6A 1H7 (CA)
(72) Inventor: Brawn, Peter Robert, Vancouver, British Columbia V6C 3R4 (CA)
(74) Representative: Maiwald Patentanwalts GmbH

(57) **Abstract**

The present invention provides an extra-oral light therapy device including an extra-oral bridge, an intra-oral tray removably connected to the extra-oral bridge, at least one extra-oral light emitting diode ("LED") array removably connected to the extra-oral bridge, and a programmable controller for controlling the extra-oral light therapy device. The present invention alternatively provides an extra-oral light therapy device including a head-set, at least one extra-oral LED array removably attached to the head-set, a connector for removably attaching the head-set to the at least one extra-oral LED array, and a programmable controller for controlling the extra-oral light therapy device. The present invention also provides an external light therapy device including a thin, molded substrate, at least one LED array mounted onto the thin, molded substrate, an attaching means for removably attaching the at least one LED array mounted onto the thin, molded substrate to the area of treatment, and a programmable controller for controlling the external light therapy device. The present invention further provides a method for treating jaw bone disorders and jaw osteonecrosis and biostimulating bone and soft tissue utilizing an extraoral light therapy device and a method for treating and stimulating soft and hard tissue and biostimulating bone utilizing an external light therapy device.

## Description

### CROSS REFERENCE TO RELATED APPLICATIONS

This application claims the benefit of U.S. provisional application No. 60/705,753, filed on August 5, 2005 and U.S. provisional application No. 60/653,828, filed on February 17, 2005.

### FIELD OF THE INVENTION

The present invention relates to a light therapy device used for the treatment of bone disorders and the biostimulation of bone and soft tissue. The present invention is designed as an external device to be used on the jaw bone or other bones and soft tissues. One or more light emitting diode ("LED") arrays are used as the means for the treatment and biostimulation.

### BACKGROUND OF THE INVENTION

Osteonecrosis is the death of bone due to inadequate blood flow to the tissues. It is known by many other names including avascular necrosis or ischemic necrosis. Ischemic necrosis literally means "dead bone from poor blood flow." It includes dead bone or bone marrow that has been slowly strangulated or nutrient-starved. It occurs because of a decrease in blood supply to specific parts of bones. The decreased circulation causes cells in the bone and bone marrow to die. Bone with chronically poor blood flow develops either a fibrous marrow; a greasy, dead fatty marrow; a very dry, sometimes leathery marrow; or a completely hollow space. Osteonecrosis is usually seen in the jaw, hips, and knees although any bone may develop this disease. There are a number of local and systemic problems capable of producing this bone disease. However, research has shown that more than 4 out of every 5 patients with osteonecrosis have a problem, usually inherited, of excessive production of blood clots in the blood vessels (See, for example, A Note to Patients with Jawbone Osteonecrosis (NICO), available at http://maxillofacialcenter.com/NICOhome.html#note).

Anything leading to blocked blood vessels can cause osteonecrosis such as abnormal red blood cells as seen in sickle cell anemia. Additionally, taking high doses of corticosteroids or expanding nitrogen bubbles (decompression sickness as seen in scuba divers) may also lead to osteonecrosis. Osteonecrosis may have no signs or symptoms, but some people experience pain, especially when pressure is applied to the bone.

Although in some cases the bone may heal itself, the majority of patients who have osteonecrosis must seek the aid of a doctor. Common treatments include curettage of the bone lesion to remove the diseased bone marrow, combining surgery with antibiotic therapy, surgery with hyperbaric chamber therapy or anticoagulation therapies.

Light therapy is a treatment option which involves stimulation of a variety of biological activities in cells and tissues that are compromised in function. Optimally functioning cells and tissues are not stimulated by light therapy. Cells and tissues contain light sensitive proteins, chromophores and cytochromes, which have the ability to absorb light energy at specific wavelengths and to transform the light energy into chemical energy. In addition, specific wavelengths stimulate enzymatic activities that are in metabolic pathways of the mitochondria, increasing cellular energy. The cells and tissues then use the chemical energy to accelerate the natural healing processes of the body. One of the most frequent effects of light therapy is increased blood and lymphatic circulation in the area exposed to the light. Other effects include decreased pain and inflammation, accelerated new bone formation, and new blood vessel formation, in addition, there is a variety of cellular and membrane activity that is stimulated by specific wavelengths and energy densities.

Light therapy treatment devices currently are used to treat tissue disorders, such as pain and inflammation. The use of a light therapy treatment device can also effectively be used to treat bone disorders, such as jaw osteonecrosis or other jaw bone disorders. Light therapy treatment devices may also be used to stimulate bone formation, soft and hard tissues, as well as for the treatment of diseased bone or tissue.

Light therapy treatment devices currently exist which use laser light and discrete light-emitting diodes or LEDs as the source of light energy. A laser uses coherent light that emits a beam of photons at specific wavelengths. An LED emits incoherent monochromatic light at specific wavelengths. The LED array has a larger surface area for treatment due to the large number of diodes on the array. The intensity of the LED array is more diffuse than laser, thereby reducing potential damage to the eye. The use of multiple wavelength LEDs on the array allows for irradiation over multiple wavelengths for greater biological activity.

Light therapy treatment may be administered by the physician, therapist or patient through the use of a hand-held light emitting wand or a light emitting device placed on the affected area of the body intended for treatment. Light emitting wands and light emitting devices are difficult to position consistently over the affected area. Sometimes a tattoo is used to identify the affected area; however, due to the difficulty in consistent placement of these designs, the constant positioning is not easily attainable. The use of a light emitting wand or a light emitting device is not an accurate, consistent or repeatable method of light therapy treatment.

Using lasers and LEDs for treatment produces significant heat due to the thermal generating nature of the lasers and LED semiconductor. Due to this production of heat, the light therapy device gets hot, making it difficult to provide effective treatment as the device loses its efficiency and safety. Due to diminished LED efficiency in response to increases in operating temperature, current light therapy devices must be reduced or pulsed in order to keep the extra-oral LED array and surface of the device cool to provide comfort to the patient and to avoid potential bums. Most LED devices are of low intensity in an attempt to correct for the heat generation. Current light therapy devices are not effective at controlling the significant heat produced.

Most currently available light therapy devices are designed for use at a physician's, dentist's or therapist's office. Light therapy treatment requires repetition in order to effectively treat jaw osteonecrosis, other jaw bone disorders, periodontitis, orthodontics, or orthopedics, to stimulate and accelerate post-oral surgery or post-periodontal surgery healing, to accelerate osseo-integration of endosseous dental implants, and to treat and stimulate new bone formation and to treat and stimulate soft and hard tissues. Thus, patients may be required to make several visits to a practitioner's office or clinic in order to complete a therapy regimen. Such repeated visits may be time consuming and/or expensive.

In view of the above, there is a need or desire for a light therapy device having the ability to apply specific wavelengths of light to affected bone for treatment and stimulation of new bone formation and/or for the treatment and stimulation of soft and hard tissues.

There is also a need or desire for a light therapy device which can produce accurate, consistent and repeatable treatment results particularly in the dental and maxillofacial areas.

There is a further need or desire for a light therapy device which can be effectively administered against the affected area without resulting in pain from the production of heat and which provides more efficient and effective light therapy treatment by correcting for this heat generation.

There is still a further need or desire for a light therapy device which can be used at home by the patient.

### SUMMARY OF THE INVENTION

In response to the challenges discussed above, a light therapy device capable of consistently and reproducibly applying specific wavelengths of light to affected bone or tissue for treatment has been developed. Suitably, the light therapy device has been developed for clinical and/or in-home use.

The extra-oral light therapy device of the present invention includes an intra-oral tray removably connected to an extra-oral bridge, at least one extra-oral light emitting diode ("LED") array removably connected to the extra-oral bridge, and a programmable controller.

In one embodiment, the extra-oral light therapy device may have a head-set style arrangement including a head-set, at least one extra-oral LED array removably connected to the head-set, a connector for removably attaching the at least one extra-oral LED array to the head-set, and a programmable controller.

In one embodiment of the present invention an external light therapy device includes at least one light emitting diode ("LED") array that is mounted on a thin, molded substrate, an attaching means for securing the external light therapy device to the area of treatment, and a programmable controller.

The present invention also relates to a method for the treatment and stimulation of soft and hard tissue and the biostimulation of bone. The method includes filing an intra-oral tray with a clear vinyl siloxane gel, inserting the intra-oral tray into a patient's mouth, allowing the vinyl siloxane gel to set thereby forming a reusable, fitted mouthpiece, connecting an extra-oral bridge to the intra-oral tray, connecting at least one extra-oral LED array to the extra-oral bridge, placing the fitted intra-oral tray into the patient's mouth, programming a controller to direct the at least one extra-oral LED array to emit pulsed or continuous incoherent monochromatic light, and emitting pulsed or continuous incoherent monochromatic light from the at least one extra-oral LED array. The controller is programmed to direct the extra-oral LED array to emit pulsed or continuous incoherent monochromatic light at predetermined rates, frequencies, intensities and durations according to a prescribed treatment regimen in order to stimulate and accelerate bone formation and healing at a select treatment area. The method may further include rotating the at least one extra-oral LED array between a sagittal axis and a vertical axis to affect the treatment area. Suitably, the extra-oral LED array emits light at wavelength between 820-890 nm and between 620-680 nm. Suitably, the programmable controller turns off the light when the level of heat produced by the light exceeds a set level.

In another embodiment, a method for treating and stimulating soft and hard tissues and biostimulating bone includes attaching at least one LED array which includes at least one reflector to a treatment area, programming a controller to direct the at least one LED array to emit pulsed or continuous incoherent monochromatic light, emitting pulsed or continuous incoherent monochromatic light from the at least one LED array onto a treatment area, and focusing the emitted light onto the treatment area using the at least one reflector, wherein the pulsed or continuous incoherent monochromatic light stimulates and accelerates bone and soft tissue formation and healing within the treatment area. Suitably, the LED array may include at least one reflector and optic which focuses the at least one LED array at an angle of about 45° to about 60°; the extra-oral LED array emits light at wavelength between 820-890 nm and between 620-680 nm; and the programmable controller turns off the light when the level of heat produced by the light exceeds a set level.

These and other embodiments are more fully described in connection with the drawings and detailed description.

### BRIEF DESCRIPTION OF DRAWINGS

FIG 1 is a front-facing view of the extra-oral light therapy device with an intra-oral tray, an extra-oral bridge, and a left and a right side extra-oral LED arrays.
FIG 2 is a right side view of the extra-oral LED array with the end of the extra-oral bridge attached to the extra-oral LED array.
FIG 3 is a front-facing, right side view of the extra-oral bridge, intra-oral tray and extra-oral LED array.
FIG 4 is a back-facing, right side view of the extra-oral bridge, intra-oral tray and extra-oral LED array.
FIG 5 is a back-facing, right side view of the extra-oral bridge, intra-oral tray and extra-oral LED array with the intra-oral tray detached.
FIG 6 is a top view of the programmable controller.
FIG 7 is a perspective view of an alternate embodiment of the extra-oral light therapy device with the head-set style arrangement, a head-set, at least one LED array, and a connector.
FIG 8 is a side view of an alternate embodiment of the extra-oral light therapy device with the head-set style arrangement, a head-set, at least one LED array, and a connector.
FIG 9 is a front-facing view of at least one LED array, and a connector detached from a head-set.
FIG 10 is a front-facing view of the external light therapy device with two LED arrays, a hinge-like member, and an attaching means.
FIG 11 is a cross-sectional view of an LED array mounted onto a substrate.
FIG 12 is a cross-sectional view of an LED array detached from a substrate.

### DETAILED DESCRIPTION OF THE INVENTION

The present invention relates to a light therapy device used for the treatment of bone disorders and the biostimulation of bone and soft tissue. The present invention is designed as an external device to be used on the jaw bone or other bones and soft tissues. One or more light emitting diode ("LED") arrays are used as the means for the treatment and biostimulation.

One embodiment of the present invention relates to a device having at least one extra-oral LED array supported by an extra-oral bridge, stabilized by an intra-oral tray and controlled by a programmable controller for the treatment of jaw osteonecrosis, other jaw bone disorders, periodontitis, orthodontics, or orthopedics, for stimulation and acceleration of post-oral surgery or post-periodontal surgery healing, and to accelerate osseo-integration of endosseous dental implants.

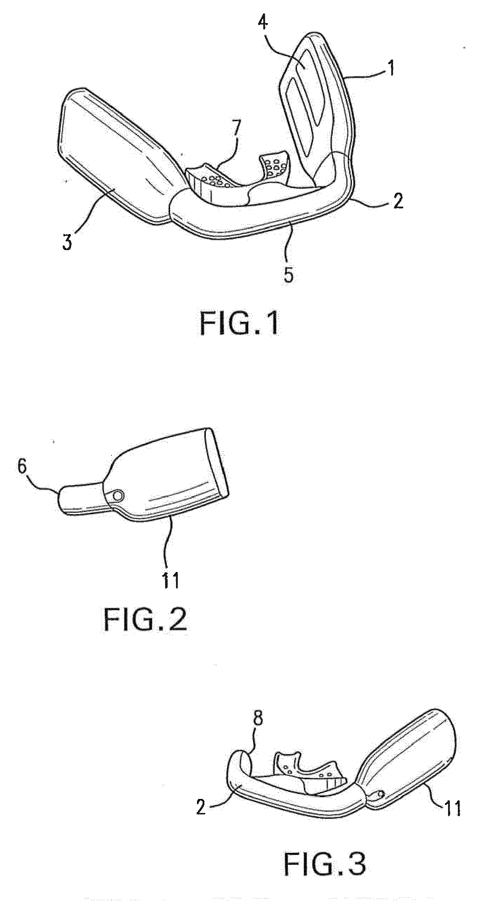

Referring to FIG 1, an extra-oral light therapy device 2 includes an extra-oral LED array 4 having a right side 1 and a left side 3, an extra-oral bridge 5, and an intra-oral tray 7.

The extra-oral bridge 5 may be removably detached from the extra-oral LED arrays and the intra-oral tray 7. Suitably, the extra-oral bridge 5 may be composed of plastic or similar material to allow for flexibility and customization of the extra-oral bridge 5 for differing patient facial morphology.

The at least one extra-oral LED array can be removably detached from the extra-oral bridge 5. FIG 2 illustrates a removably detached extra-oral LED array right side 1.

Suitably, the extra-oral bridge 5, the extra-oral LED array right side 1, and the extra-oral LED array left side 3 may be secured together via a connector. For example, the extra-oral bridge 5, the extra-oral LED array right side 1, and the extra-oral LED array left side 3 may be connected by inserting the male portion 6 of the extra-oral LED array right side 1 and the extra-oral LED array left side 3 into the female portions 8 of the extra-oral bridge 5 as shown in FIG 1. Suitably, the connector which joins the extra-oral bridge 5, the extra-oral LED array right side 1, and the extra-oral LED array left side 3 allows the extra-oral LED array right side 1 and the extra-oral LED array left side 3 to be detached for ease of use and flexibility as shown in FIG 2.

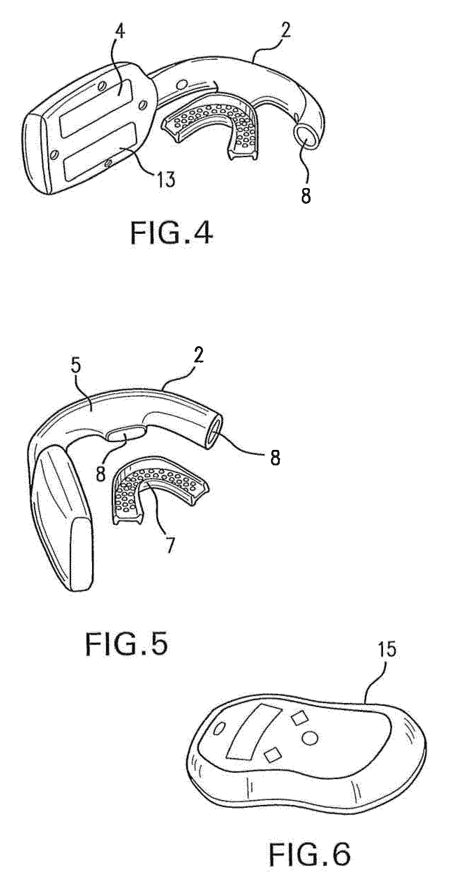

The extra-oral LED array right side 1 is further comprised of an outer surface 11 as shown in FIG 3 and an inner surface 13 as shown in FIG 4. The inner surface 13 of the extra-oral LED array right side 1 is the surface that is placed against the area of treatment. The direction of light emitted is from the inner surface 13 towards the area of treatment. FIG 3 and FIG 4 illustrate the extra-oral LED array right side 1 only for purposes of illustration of at least one extra-oral LED array.

The extra-oral bridge 5 houses the intra-oral tray 7. The intra-oral tray 7 may be connected to the extra-oral bridge 5 by inserting a male portion 6 of the intra-oral tray 7 into a female portion 8 of the extra-oral bridge 5, as illustrated in FIG 5. The intra-oral tray 7 is intended for insertion into a patient's mouth and is suitably shaped to fit around a patient's full set of teeth for better stability. Suitably, the intra-oral tray 7 is removably attached to the extra-oral bridge 5 in order to allow the physician or dentist to dispose of the intra-oral tray 7 after use, therefore resulting in a more hygienic oral light therapy treatment. In one embodiment, the intra-oral tray 7 may be composed of perforated plastic or other similarly flexible material. Prior to extra-oral light therapy treatment, the intra-oral tray 7 may be filled with a clear vinyl siloxane gel or similar material which sets and allows exact alignment of the intra-oral tray 7 and consistent targeting of the affected oral bone during subsequent treatments. During the extra-oral light therapy treatment, the patient bites onto the intra-oral tray 7 to stabilize the extra-oral light therapy device 2. The consistent alignment and targeting of the affected oral bone during subsequent treatments creates repeatable treatments which further stimulates and accelerates the treatment of jaw osteonecrosis, other jaw bone disorders, periodontitis, orthodontics, or orthopedics, stimulates and accelerates post-oral surgery or post-periodontal surgery healing, and accelerates osseo-integration of endosseous dental implants.

The extra-oral LED array right side 1 and the extra-oral LED array left side 3 may be constructed of integrated thick-film ceramo-metal LED wafers, thin conductive PCB with a plurality of LEDs or similar thermally conductive LED wafers with a metal substrate, which efficiently transfers the heat from the LEDs to an underlying pin-fin aluminum heat sink, copper heat sink, or similar thermally conductive heat sink (not shown). The LEDs can be arranged closely due to the heat efficiency of the device.

The extra-oral LED array right side 1 and the extra-oral LED array left side 3 are comprised of an LED array which emits incoherent monochromatic light at varying frequencies and high intensity wavelengths. The light energy emitted from the LED array may be continuous or pulsed at predetermined rates and frequencies. Clusters of high-powered discrete LEDs and other high-powered LED arrays may be utilized with forced air or liquid cooling methods of thermal cooling. This allows for treatment without the danger of potential bums to the patient and allows for greater efficiency and control of the device. The LEDs are arranged in a variety of patterns to achieve uniform optical density on the treatment area. The LEDs are suitably arranged in staggered parallel rows to maximize the number of LEDs on the LED array. The use of an LED array is advantageous due to its ability to cover a larger surface area, its greater intensity, and its larger wavelength, which allows the irradiation to cover a wider spectrum for greater biological activity. Suitably, the LED array may emit light at wavelengths of between about 820 to about 890 nm and between about 620 to about 680 nm. The use of an LED array is also advantageous as it has been shown to effectively stimulate and accelerate affected oral bone formation and healing in a wider treatment area. Suitably, the LED array may be rotated between a sagittal axis (not shown) and a vertical axis (not shown) which results in the ability to better target the affected oral bone.

FIG 6 illustrates the programmable controller 15. The programmable controller 15 may be composed of a microprocessor and the associated electronic circuitry and suitably powers the present invention. Suitably, the circuitry in the programmable controller 15 monitors the changes in current/voltage and calculates the temperatures of the LED by using an algorithm programmed into the programmable controller software. A fail safe circuitry will shut off the current and light if the heat exceeds a pre-set level. A physician, dentist, or therapist may program a patient's treatment regimen into the programmable controller 15. The programmable controller 15 may control the energy density, pulse frequency and/or duration of light emitted by the extra-oral light therapy device 2. The programmable controller 15 may have pre-set programs built in, pre-defined by the physician, dentist, or therapist so that a patient is able to use the device under specific pre-programmed instructions. A patient can then utilize the extra-oral light therapy device 2 at home through the use of the programmed treatment regimen in the programmable controller 15. The programmable controller 15 may be a separate, remote unit or may be directly connected to the present invention.

During extra-oral light therapy treatment, the intra-oral tray 7 is preferably placed in a patient's mouth to provide stability. The extra-oral bridge 5 preferably conforms around the jaw line of a patient. The extra-oral LED array right side 1 and extra-oral LED array left side 3 are positioned on the right and left side of a patient's jaw line, respectively. The physician, dentist, or therapist at his office or a patient at his home then performs the prescribed extra-oral light therapy treatment on the affected oral bone resulting in the treatment of jaw osteonecrosis, other jaw bone disorders, periodontitis, orthodontics, or orthopedics, stimulation and acceleration of post-oral surgery or post-periodontal surgery healing, and acceleration of osseo-integration of endosseous dental implants.

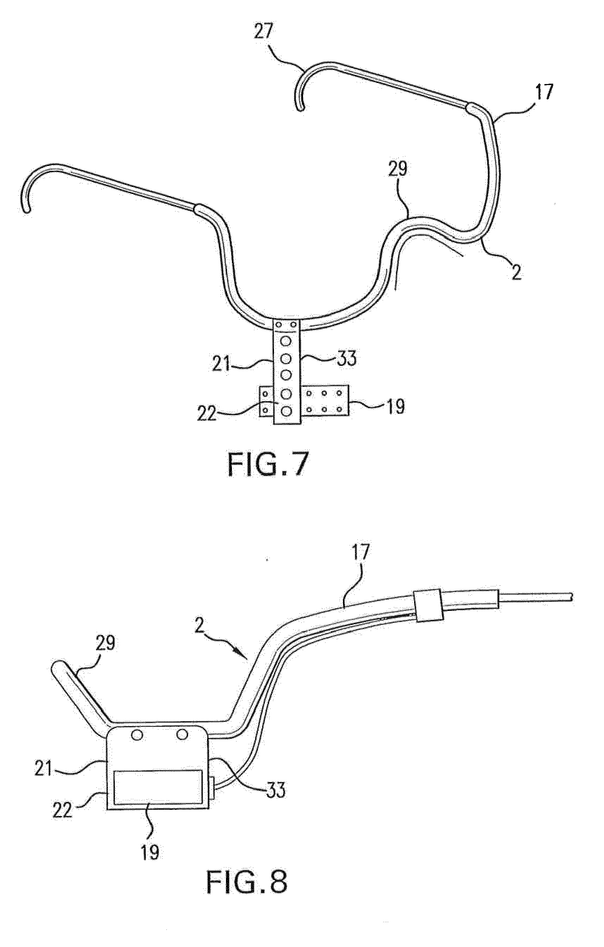

In another embodiment, as shown in FIG 7 and FIG 8, the extra-oral light therapy device 2 may have a head-set style arrangement. The extra-oral light therapy device 2 includes a head-set 17, at least one extra-oral LED array 19, and connector 21. The head-set 17 can be modeled as a traditional pair of eyeglasses with form-fitting arms 27 that fit above and around the ears, and a frame 29 that fits on the bridge of the nose to secure the pair of glasses. The form-fitting arms 27 can be made of any firm, resilient material that allows for some flexibility for a better and more secure fit for individual users. The form-fitting arms 27 can also be adjusted horizontally along their axis. The frame 29 can also be adjustable to allow for a better and more secure fit. The head-set 17 may also include lenses (not shown) like a traditional pair of eyeglasses. Suitably, the lenses may be made of a protective material to shield the patient's eyes from the LED array.

In another embodiment, the head-set 17 can be modeled as an adjustable strap (not shown) which fits around the crown of a patient's head for securing the extra-oral light therapy device 2. The adjustable strap can also fit around a patient's chin and extend back to the crown and around the crown of a patient's head. The adjustable strap is preferably made of a flexible, elastic woven material.

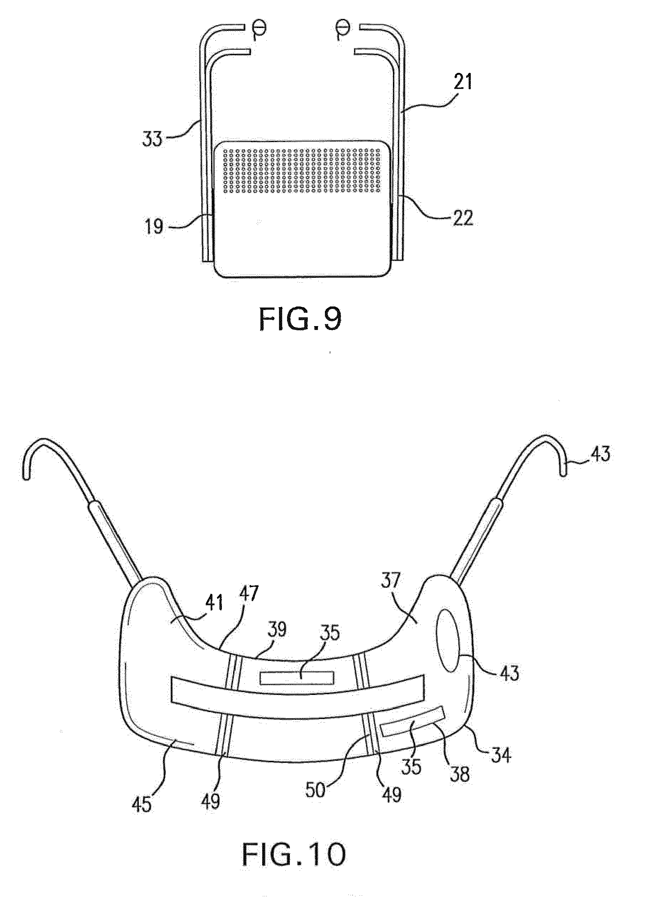

A connector 21 is attached to the head-set 17. A bar, rod or similar device 33 is fastened to the connector 21. The at least one extra-oral LED array 19 is then attached through a clip or similar mechanism 22 to the bar, rod or similar device 33. As shown in FIG 9, the at least one extra-oral LED array 19 can be removably detached from the head-set 17. The at least one extra-oral LED array 19 may be adjusted along a horizontal axis (not shown), relative to the head-set 17, or a vertical axis (not shown), relative to the head-set 17. The bar, rod, or similar device 33 may be comprised of a flexible but firm material sufficient to sustain the weight of the at least one extra-oral LED array 19.

The present invention also relates to a method of treatment for jaw osteonecrosis, other jaw bone disorders, periodontitis, orthodontics, or orthopedics, a method of stimulation and acceleration of post-oral surgery or post-periodontal surgery healing, and a method of acceleration of osseo-integration of endosseous dental implants. The method utilizes the extra-oral light therapy device 2. Prior to extra-oral light therapy treatment, the intra-oral tray 7 is preferably filled with a clear vinyl siloxane gel or similar material which sets and allows exact alignment of the intra-oral tray 7 and consistent targeting of the affected oral bone during subsequent treatments. The intra-oral tray 7 is connected to the extra-oral bridge 5 and the at least one extra-oral LED array 19 is connected to the extra-oral bridge 5. The intra-oral tray 7 is inserted into a patient's mouth and is preferably shaped to fit around a patient's full set of teeth for better stability. A physician, dentist, or therapist programs a patient's prescribed treatment regimen into the programmable controller 15. The programmable controller 15 controls the energy density, pulse frequency and duration of the extra-oral light therapy device 2. The programmable controller 15 runs a patient's prescribed treatment regimen causing the at least one extra-oral LED array to emit pulsed or continuous incoherent monochromatic light at the prescribed rates and frequencies onto the treatment area. Therefore, stimulating and accelerating bone formation and healing at a patient's treatment area for the treatment of jaw bone disorders and jaw osteonecrosis.

Another embodiment of the present invention relates to an external light therapy device 34 comprising at least one LED array 35 that is mounted on a thin, molded substrate 51, an attaching means 43 for securing the device to the area of treatment, and a programmable controller 15 for the treatment and stimulation of soft and hard tissue and the biostimulation of bone.

Referring to FIG 10, the external light therapy device 34 having a right section 37, a center section 39 and a left section 41 includes an LED array 35. FIG 10 illustrates the present invention for use for the treatment and stimulation of the jaw and facial bones and tissues, and fits around a patient's mouth, with the right section 37 and the left section 41 secured on the right and left sides of a patient's face with the attaching means 43. The attaching means 43 can be an adhesive such as double-sided adhesive tape or the attaching means 43 can also be form-fitting arms which surround the ear, such as those used in traditional eyeglasses, wherein such attaching means are designed for use in patients of differing facial sizes and to allow for flexibility for a more comfortable patient fit. Alternatively, the attaching means 43 can be utilized as an intra-oral means such as bite tabs or a tray device for proper positioning.

When using the external light therapy device 34 for treatment and stimulation of other bone or soft tissues, such as the hip, the device can be attached to the treatment area with use of an adhesive such as double-sided adhesive tape (not shown). Alternatively, the external light therapy device 34 can be placed or sewn into a pouch, undergarment or similar garment and attached to the treatment area through means of a strap, button or similar attaching means (not shown).

The external light therapy device 34 as shown in FIG 10 has at least one LED array 35 which is preferably permanently mounted on a thin, molded substrate 51. More than one LED array may be used in the device. For example, FIG 10 shows the device with two (2) arrays. The LED arrays may be arranged such that there is a lower level 45 and an upper level 47. The LED arrays may be removably attached through the use of one or more connectors 38 such as ribbon connectors. In between the LED arrays, a hinge-like member 49 is preferably integrated to allow for a more secure fit around the facial area. The hinge-like member 49 may be a thin crease 50 set into the substrate material, as illustrated in FIG 10. The hinge-like member 49 allows the center section 39 to fit around a patient's mouth and the right section 37 and the left section 41 to fit around a patient's face.

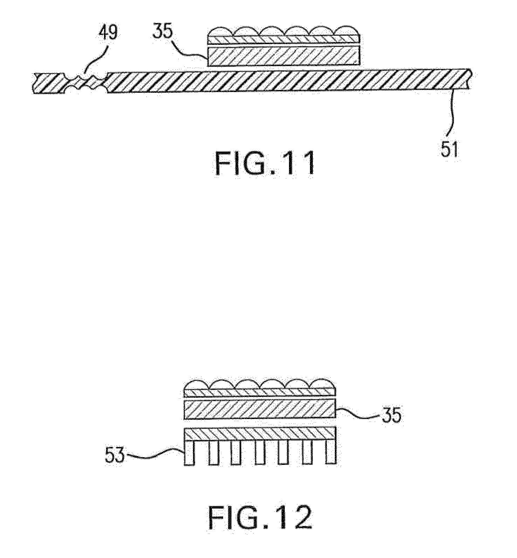

The at least one LED array 35 may be permanently mounted on a thin, molded substrate 51 as illustrated in FIG 11. FIG 12 shows a cross-section of the at least one LED array 35 of the external light therapy device 34 detached from the substrate 51. A clip or similar attaching means 53 allows the at least one LED array 35 to be mounted onto the substrate 51. The thin, molded substrate 51 is used as a heat sink as described above. The substrate 51 may be made of aluminum, copper or similar thermally conductive material to allow for a flexible, but somewhat rigid material. Optical focus from the LED array may be built into the array; reflectors and optics may be included as part of the LED array and these are suitably encapsulated in plastic or similar material and act to direct the light from the LED array. The optimal optical focus for the reflectors is approximately at an angle between 45-60°.

The at least one LED array 35 emits incoherent monochromatic light at varying frequencies and high intensity wavelengths. The light energy emitted from the LED array may be continuous or pulsed at predetermined rates and frequencies. The LEDs are arranged in a variety of patterns to achieve uniform optical density on the treatment area. The LEDs are suitably arranged in staggered parallel rows to maximize the number of LEDs on the LED array. Suitably, the LED array may emit light at wavelengths of between about 820 to about 890 nm and between about 620 to about 680 nm.

FIG 6 illustrates the programmable controller 15 as described above.

The present invention also relates to a method for the treatment and stimulation of soft and hard tissue and the biostimulation of bone. The at least one LED array 35 is first attached to the desired area of treatment. A physician, dentist, or therapist programs a patient's prescribed treatment regimen into the programmable controller 15. The programmable controller 15 controls the energy density, pulse frequency and duration of the light emitted from the external light therapy device 34. The programmable controller 15 runs a patient's prescribed treatment regimen causing the at least one LED array 35 to emit pulsed or continuous incoherent monochromatic light at the predetermined rates and frequencies onto the treatment area. The light therapy device features provide effective, stabilized, repeatable, accurate, programmable, and consistent light therapy for the treatment and stimulation of soft and hard tissue and the biostimulation of bone.

While in the foregoing specification this invention has been described in relation to certain preferred embodiments thereof, and many details have been set forth for the purpose of illustration, it will be apparent to those skilled in the art that the invention is susceptible to additional embodiments and that certain details described herein can be varied considerably without departing from the basic principles of the invention.

According to a further aspect, the following examples (e) are provided:
e1) An extra-oral light therapy device for the treatment of jaw bone disorders and jaw osteonecrosis and biostimulation of bone and soft tissue, comprising:
   - an extra-oral bridge;
   - an intra-oral tray removably connected to the extra-oral bridge;
   - at least one extra-oral light emitting diode ("LED") array removably connected to the extra-oral bridge; and
   - a programmable controller for controlling the extra-oral light therapy device.
e2) The extra-oral light therapy device of example e1) wherein the at least one extra-oral LED array comprises an extra-oral LED array right side and an extra-oral LED array left side.
e3) The extra-oral light therapy device of example e2) wherein the extra-oral bridge comprises three female portions for receiving male portions of the extra-oral LED array right side, the extra-oral LED array left side, and the intra-oral tray.
e4) The extra-oral light therapy device of example e1) wherein the extra-oral bridge is flexibly molded to allow customization for different facial morphology.
e5) The extra-oral light therapy device of example e1) wherein the at least one extra-oral LED array comprises an outer surface and an inner surface wherein the inner surface emits light toward an area of treatment.
e6) The extra-oral light therapy device of example e1) wherein the at least one extra-oral LED array rotates between a sagittal axis and a vertical axis for treatment accuracy.
e7) The extra-oral light therapy device of example e1) wherein the at least one extra-oral LED array comprises a plurality of thick-film ceramo-metal LED wafers and a metal substrate for transferring heat produced to an aluminum heat sink.
e8) The extra-oral light therapy device of example e1) wherein the programmable controller is programmed with a patient's prescribed treatment regimen.
e9) An extra-oral light therapy device for the treatment of jaw bone disorders and jaw osteonecrosis and biostimulation of bone and soft tissue, comprising:
   - a head-set;
   - at least one extra-oral LED array removably attached to the head-set;
   - a connector for removably attaching the head-set to the at least one extra- oral LED array; and
   - a programmable controller for controlling the extra-oral light therapy device.
e10) The extra-oral light therapy device of example e9) wherein the head-set comprises form-fitting arms that conform to a patient's ears and a frame that secures the head-set to a patient's nose.
e11) The extra-oral light therapy device of example e9) wherein the connector removably attaches the at least one extra-oral LED array to the head-set by clipping the at least one extra-oral LED array to a bar and rod fastened to the connector.
e12) The extra-oral light therapy device of example e9) wherein the at least one extra-oral LED array adjusts along a horizontal axis and a vertical axis.
e13) The extra-oral light therapy device of example e9) wherein the at least one extra-oral LED array comprises a plurality of thick-film ceramo-metal LED wafers and a metal substrate for transferring heat produced to an aluminum heat sink.
e14) The extra-oral light therapy device of example e9) wherein the programmable controller is programmed with a patient's prescribed treatment regimen.
e15) An external light therapy device for the treatment and stimulation of soft and hard tissue and the biostimulation of bone, comprising:
   - a thin, molded substrate;
   - at least one LED array mounted onto the thin, molded substrate;
   - an attaching means for removably attaching the thin, molded substrate to a treatment area; and
   - a programmable controller for controlling the external light therapy device.
e16) The external light therapy device of example e15) wherein the external light therapy device comprises a right section, a center section, and a left section.
e17) The external light therapy device of example e16) wherein the external light therapy device comprises a hinge-like member between the right section and center section and a hinge-like member between the left section and center section for aligning along a patient's face.
e18) The external light therapy device of example e16) wherein the attaching means is double-sided adhesive tape or form-fitting arms for securing the right section and left section onto the patient's face and ears, respectively.
e19) The external light therapy device of example e15) wherein the external light therapy device further comprises a lower level and an upper level for removably attaching the at least one LED array to the thin, molded substrate.
e20) The external light therapy device of example e15) wherein the programmable controller is programmed with a patient's prescribed treatment regimen.
e21) The external light therapy device of example e15) wherein the at least one LED array is sewn, strapped or buttoned into a pouch, garment, or undergarment.
e22) The external light therapy device of example e15) wherein the thin, molded substrate comprises an aluminum heat sink for absorbing heat produced by the at least one LED array.
e23) The external light therapy device of example e15) wherein the at least one LED array further comprises optics to direct the light emitted from the LED array.
e24) A method for treating jaw bone disorders and jaw osteonecrosis and biostimulating bone and soft tissue comprising the steps of:
   - filling an intra-oral tray with a clear vinyl siloxane gel;
   - inserting the intra-oral tray into a patient's mouth;
   - setting the clear vinyl siloxane gel around the patient's teeth to form a fitted intra-oral tray;
   - connecting the fitted intra-oral tray to the extra-oral bridge;
   - connecting the at least one extra-oral LED array to the extra-oral bridge;
   - placing the fitted intra-oral tray into the patient's mouth for consistent alignment during treatment;
   - programming a controller to direct the at least one extra-oral LED array to emit pulsed or continuous incoherent monochromatic light; and
   - emitting pulsed or continuous incoherent monochromatic light from the at least one extra-oral LED array onto a treatment area;
      wherein the controller is programmed to direct the extra-oral LED array to emit pulsed or continuous incoherent monochromatic light at predetermined rates, frequencies, intensities and durations according to a prescribed treatment regimen in order to stimulate and accelerate bone formation and healing at a select treatment area.
e25) The method of example e24) wherein the at least one extra-oral LED array emits light between 820- 890 nm and 620- 680 nm.
e26) The method of example e24) further comprising rotating the at least one extra-oral LED array between a sagittal axis and a vertical axis to affect the treatment area.
e27) The method of example e24) wherein the programmable controller turns off the light when the level of heat produced by the light exceeds a set level.
e28) A method for treating and stimulating soft and hard tissue and biostimulating bone comprising the steps of:
   - attaching at least one LED array to a treatment area;
   - programming a controller to direct the at least one LED array to emit pulsed or continuous incoherent monochromatic light; and
   - emitting pulsed or continuous incoherent monochromatic light from the at least one LED array onto the treatment area;
      wherein the controller is programmed to direct the extra-oral LED array to emit pulsed or continuous incoherent monochromatic light at predetermined rates, frequencies, intensities and durations according to a prescribed treatment regimen in order to stimulate and accelerate bone formation and healing at a select treatment area.
e29) The method of example e28) wherein the at least one LED array emits light approximately between 820- 890 nm and 620- 680 nm.
e30) The method of example e28) wherein the at least one LED array comprises optics which focus the light emitted from the at least one LED array at an angle approximately between 45- 60°.
e31) The method of example e28) wherein the programmable controller turns off the light when the level of heat produced by the light exceeds a set level.

## Claims

1. An extra-oral light therapy device (2) for use in orthodontic treatment, comprising:
- an extra-oral support (5), the extra-oral support comprising a head set (17) and a frame (29) adapted to fit on the bridge of a patient's nose and to lie over the patient's face when the device is worn by the patient;
- at least one extra-oral light emitting diode ("LED") array (19) that is removably attached to the frame via a connector (21), and that is adapted to irradiate light through the patient's face to the patient's jawbone and soft tissue when the device is worn by the patient and in use; and
- a programmable controller (15) for controlling the extra-oral light therapy device.

2. The extra-oral light therapy device of claim 1, wherein the head-set comprises form-fitting arms (27) that conform to a patient's ears.

3. The extra-oral light therapy device of claim 1, wherein the connector removably attaches the at least one extra-oral LED array (19) to the frame by clipping the at least one extra-oral LED array to a bar or rod (33) fastened to the connector.

4. The extra-oral light therapy device of claim 1, wherein the at least one extra-oral LED array adjusts along a horizontal axis relative to the headset and a vertical axis relative to the headset.

5. The extra-oral light therapy device of claim 1, wherein the programmable controller comprises a fail-safe circuitry that shuts off current and light if heat in the device exceeds a preset level.

6. The extra-oral light therapy device of claim 1, wherein the programmable controller is arranged to direct the at least one extra-oral LED array to emit light at a predetermined rate, frequency, energy density, intensity and duration according to a patient's prescribed treatment regimen.

7. The extra-oral light therapy device of claim 1, wherein the at least one extra-oral LED is capable of emitting light at wavelengths between about 620 to about 680 nm and between about 820 to about 890 nm.

8. The extra-oral light therapy device of claim 1, further comprising thermal cooling components comprising a heat sink (51), the thermal cooling components being configured for forced air or liquid cooling.

9. The external light therapy device of claim 1, wherein the at least one extra-oral LED array further comprises optics or reflectors encapsulated in plastic that are capable of directing the light emitted from the at least one extra-oral LED array.

10. The extra-oral light therapy device of claim 1, wherein the head set comprises an adjustable strap adapted to fit around the crown of the patient's head for securing the extra-oral light therapy device

11. The extra-oral light therapy device of claim 1, comprising a plurality of extra-oral LED arrays.

12. The extra-oral light therapy device of claim 1, wherein the at least one extra-oral LED array rotates between a sagittal axis and a vertical axis.

13. The extra-oral light therapy device of claim 12, wherein the at least one LED array comprises one or more LEDs.

14. The extra-oral light therapy device of claim 2, wherein the form-fitting arms (27) are adapted to fit above and around the patient's ears without covering the ears.

15. The extra-oral light therapy device of claim 1, wherein the at least one extra-oral LED array is attached by inserting a male portion (6) of the at least one extra-oral LED array into a female portion (8) of the frame.

16. The extra-oral light therapy device of claim 1, wherein the frame allows for the at least one extra-oral LED array to be attached at more than one position relative to the frame.

17. The extra-oral light therapy device of claim 1, wherein the at least one extra-oral LED array irradiates light in an amount that is effective for the orthodontic treatment.

18. The extra-oral light therapy device of claim 1, wherein the at least one extra-oral LED array is adapted to contact a side of the patient's face when the device is worn by the patient

19. The extra-oral light therapy device of claim 1, further comprising another extra-oral LED array, wherein one extra-oral LED array is adapted to contact the right side of the patient's face when the device is worn by the patient, and the other extra-oral LED array is adapted to contact the left side of the patient's face when the device is worn by the patient.

20. The extra-oral light therapy device of claim 16, wherein a hinge-like member is integrated in the head set between the plurality of extra-oral LED arrays.
